# EUROPEAN PATENT APPLICATION

(11) **EP 3 929 307 A1**
(43) Date of publication of application: **29.12.2021**
(21) Application number: 20382550.0
(22) Date of filing: 23.06.2020
(51) Int. Cl.: C12Q 1/6823, C12Q 1/6883

(54) **METHODS FOR DETECTING THE PRESENCE OF SEPSIS**

(71) Applicant: SOMAprobes SL, 20009 San Sebastián (ES)
(72) Inventor: Hernandez, Luiza I., 20009 Donostia-San Sebastian (ES); Hernández, Frank J., 20009 Donostia-San Sebastián (ES)
(74) Representative: Hoffmann Eitle

(57) **Abstract**

The present invention shows that the measurement of the inhibitory activity of a subject's RBCs-RI (Red Blood Cells cytosolic ribonucleic inhibitors) on the blood/serum RNases is a novel way of differentiating healthy from septic blood and represents a valid biomarker with great clinical potential. In particular, the effect of lysing the erythrocytes of a blood biological sample and the subsequent release of the cytosolic ribonuclease inhibitors (RI), in particular the Blood ERythrocyte-derived RNase Inhibitors (BERRI), provides for a significant decrease in RNAse activity in control or uninfected samples, while little effect is observed in septic samples, showing that the reduction of nuclease activity in the control or uninfected samples is due to the specific inhibition, by the BERRI, of the blood/serum RNases, in particular of the RNAse A type endonuclease.

## Description

### Technical field of the invention

The present invention pertains to the medical field, in particular to the discovery of a biomarker and methods for detecting the presence of sepsis in a biological sample by measuring the nuclease activity of said sample.

### Background of the invention

Sepsis is defined by the Third International Consensus Definitions for Sepsis and Septic Shock (Sepsis-3) as "a life-threatening organ dysfunction caused by a dysregulated host response to infection".¹ If not recognized early and managed promptly, it can lead to septic shock, multiple organ failure and death. Any type of infectious pathogen can potentially cause sepsis. Antimicrobial resistance is a major factor determining clinical unresponsiveness to treatment and rapid evolution to sepsis and septic shock. Sepsis patients with resistant pathogens have been found to have a higher risk of hospital mortality. Anyone affected by an infection can progress to sepsis conditions but some vulnerable populations such as elderly people, pregnant women, neonates, hospitalized patients, and people with HIV/AIDS, liver cirrhosis, cancer, kidney disease, autoimmune diseases and no spleen, are at higher risk.² Septic shock is the deadliest form of sepsis, resulting in higher mortality rates. Sepsis is frequently under-diagnosed at an early stage, when is still potentially reversible. Sepsis is treatable, and timely implementation of targeted interventions improves outcomes. Unfortunately, diagnosis of sepsis remains a major challenge for many reasons. Sepsis diagnosis still relies on nonspecific physiological criteria (including changes in temperature and heart and respiration rates) and culture-based pathogen detection. This results in diagnostic uncertainty, therapeutic delays, the misuse and overuse of antibiotics and many other problems that increase mortality.^{3,4}

Furthermore, in the case of patients suspected of sepsis, clinical microbiology tests may be negative but when positive often require two or more days to produce actionable results. These microbiologic data suffer from significant numbers of false-positives and false-negatives when attempting to identify the actual microbial cause of sepsis.^{5,6} Importantly, in clinical practice, only 14-30% of sepsis cases are clinical-relevantly diagnosed by blood culture. Thus, early diagnosis of sepsis is critical because intervention could potentially have the greatest patient benefit early in the disease course. Antibiotic administration decreases the likelihood of death by 7.6% per hour.⁷ Unfortunately, standard diagnostic tests are not available within this critical time frame to allow focused, effective, and potentially life-saving medical interventions.

Recently, biochemical tests (immunoassays and molecular methods), especially, PCR-based approaches are gaining popularity as they are fast and very sensitive. However, they typically require a positive blood culture and the species and strain-specific primers that may or may not be available for a particular organism. Other faster adjunct standard hematological analyses used in routine clinical practice have low sensitivity and specificity, particularly in neonatal patients. Recently, biomarkers such as C-reactive protein (CRP), procalcitonin (PCT), and the neutrophil marker CD64 have made their way into sepsis evaluations, with somewhat limited success.

This underscores the need for novel types of biomarkers that could in theory characterize the host response to rule in/out infection, and to more accurately distinguish patients who are truly septic or at risk of becoming septic and assist with the appropriate use of antibiotics.

### Brief description of figures of the invention

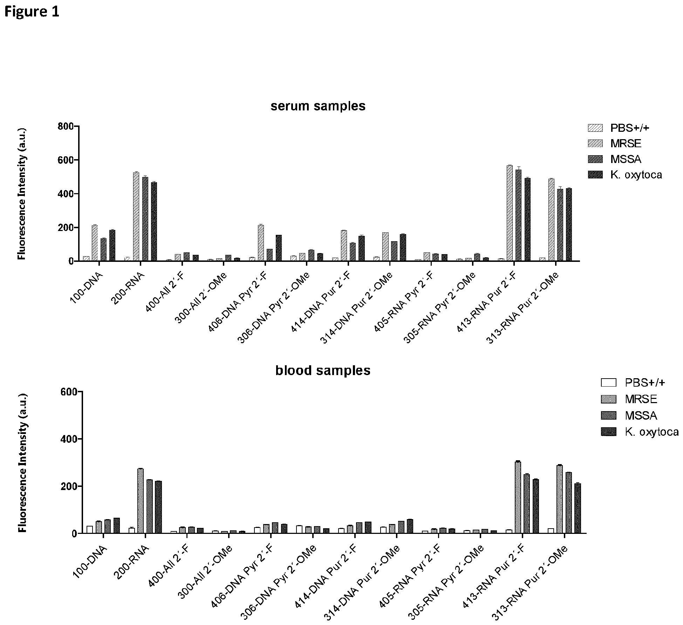

**Figure 1****:** First round of screening of patient blood and serum samples using 12 probes library. Nuclease activity was measured by fluorescence in paired blood and serum patient samples infected with *Methicillin resistant staphylococcus epidermidis* (MRSE), *Methicillin sensitive staphylococcus aureus* (MSSA) and *Klebsiella oxytoca.* The samples were screened using a library of 12 probes, containing either all naked DNA or RNA sequences or sequences harboring chemical modifications at the 2'position of the sugar ribose (2'- O methyl and 2'-fluoro).

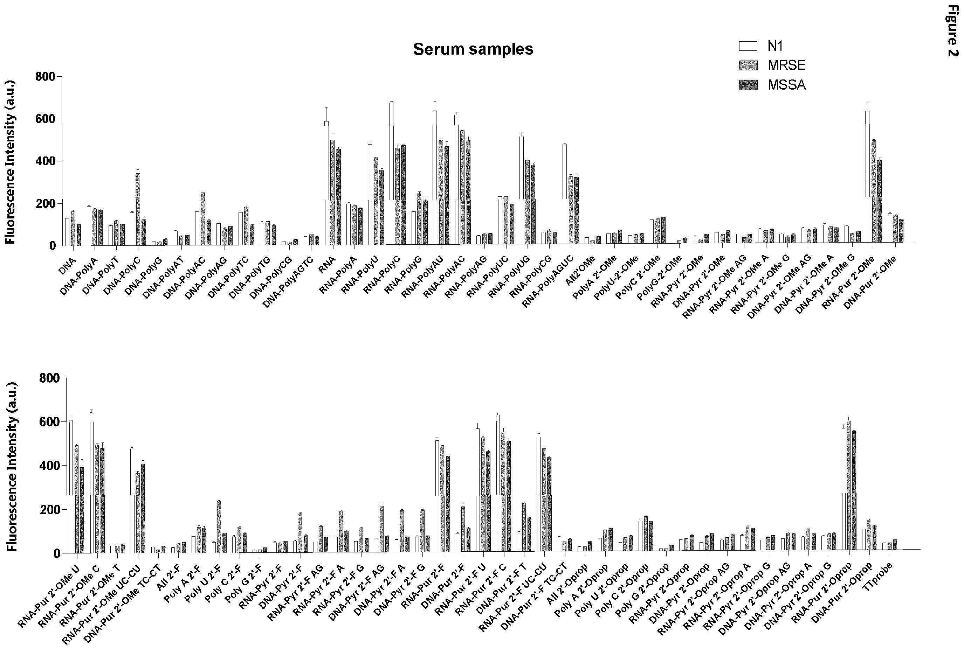

**Figure 2****:** Second round of screening of serum samples using 80 probes library. Nuclease activity was measured in healthy (N1) or MRSE/MSSA infected blood serum using the extended library containing the 80 nucleic acid probe sequences of Table 1.

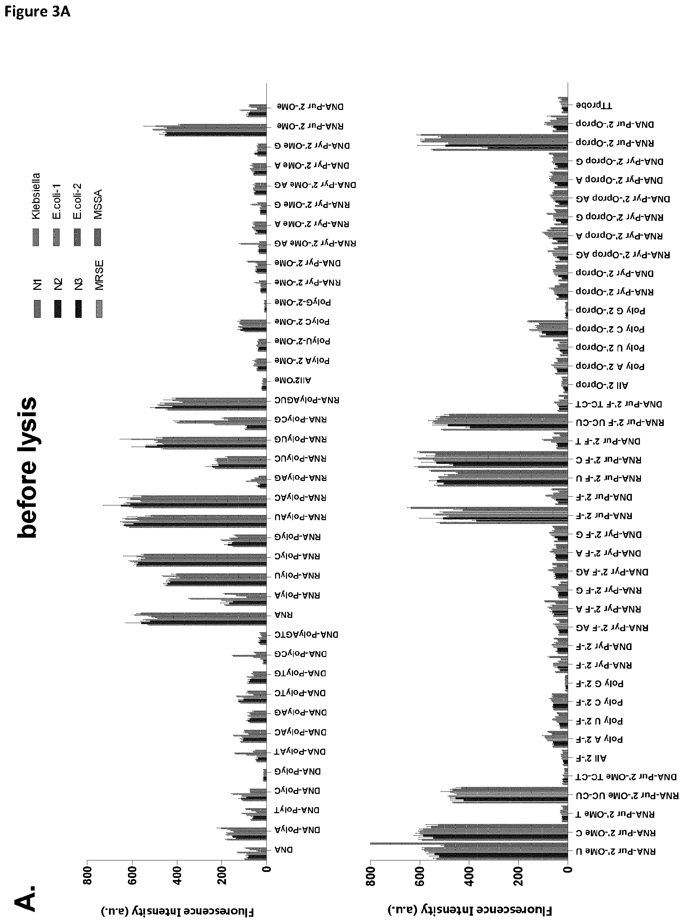

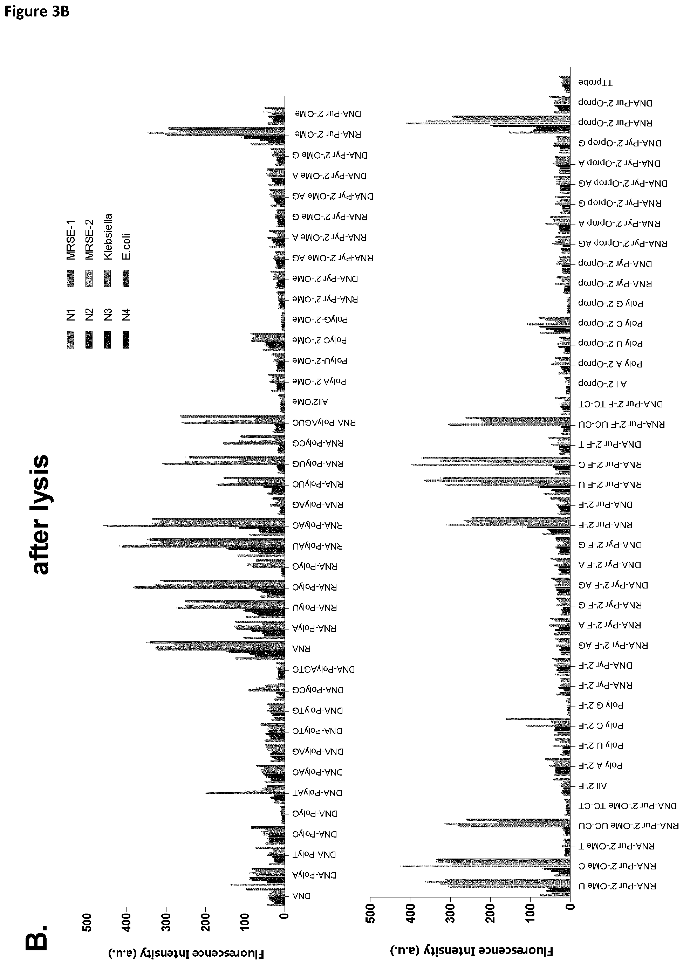

**Figure 3****:** Second round of screening of blood from healthy (control) donors and infected blood samples from patients using 80 probes library. Healthy (N1-3, in blue color) or pathogen infected blood samples (infected with *MRSE, Klebsiella, E. coli-1, E. coli-2* or *MSSA)* were screened for nuclease activity, measured by fluorescence, before (Figure 3A) or after (Figure 3B) blood lysis using the extended library containing the 80 nucleic acid probe sequences of Table 1.

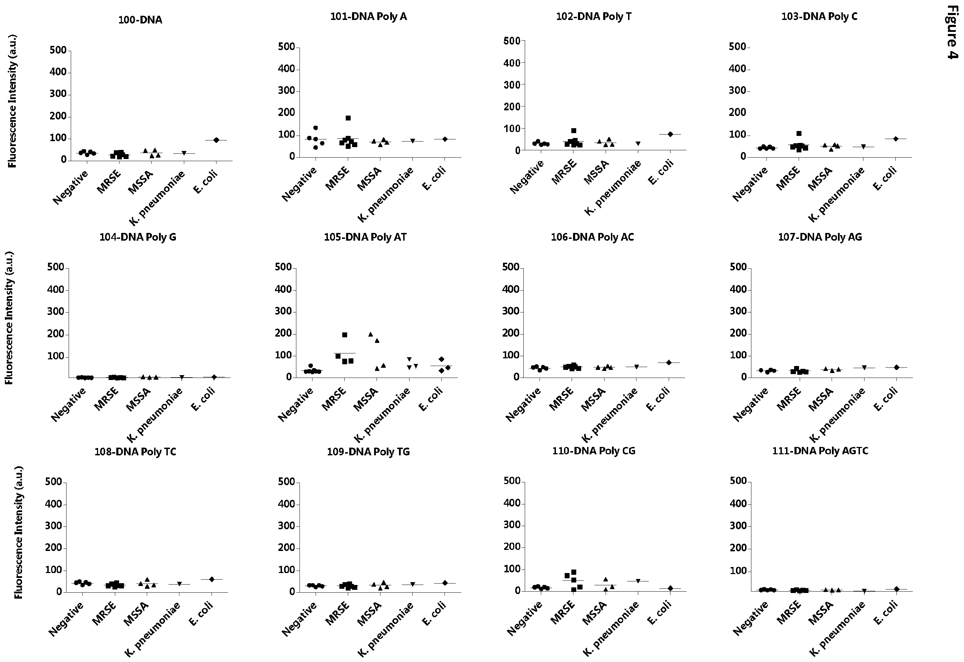

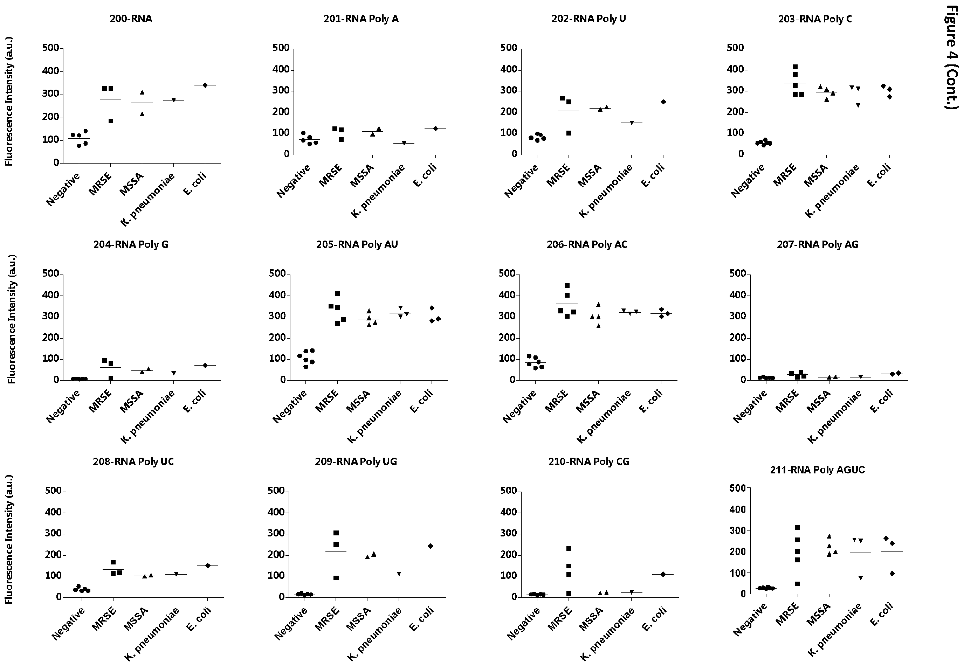

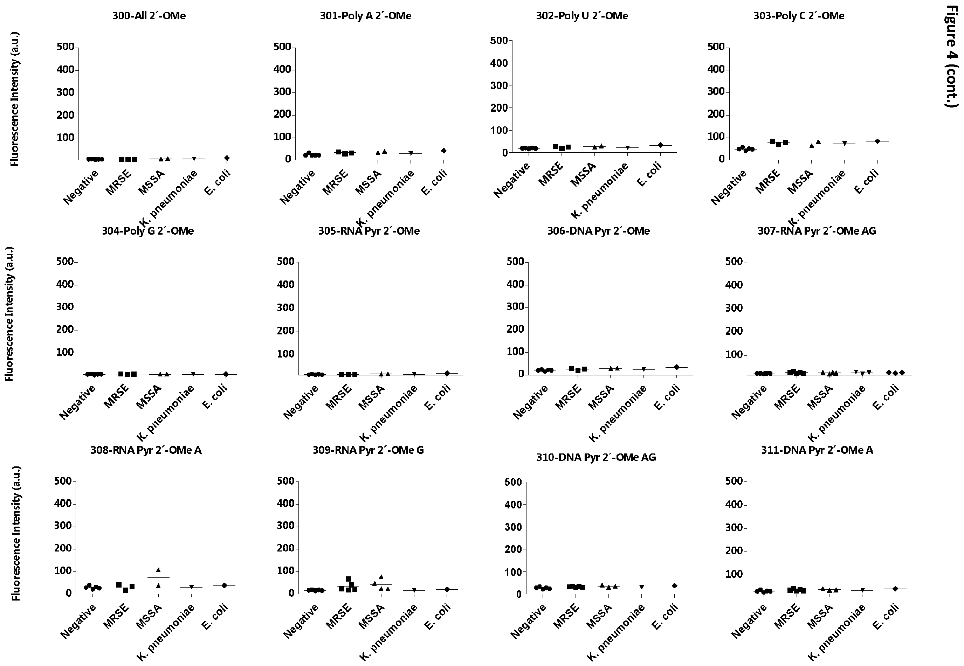

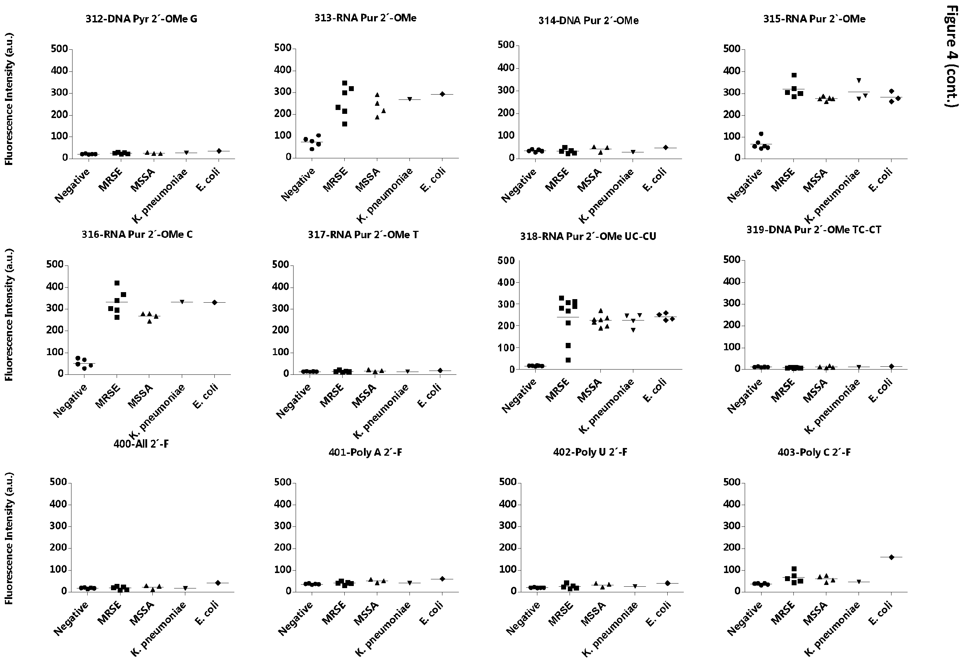

**Figure 4****:** Probes performance for discriminating between healthy (control) and infected blood samples. Fluorescence intensity values correspond to the degradation efficiency of the nucleic acid probe substrate by the blood sample nucleases.

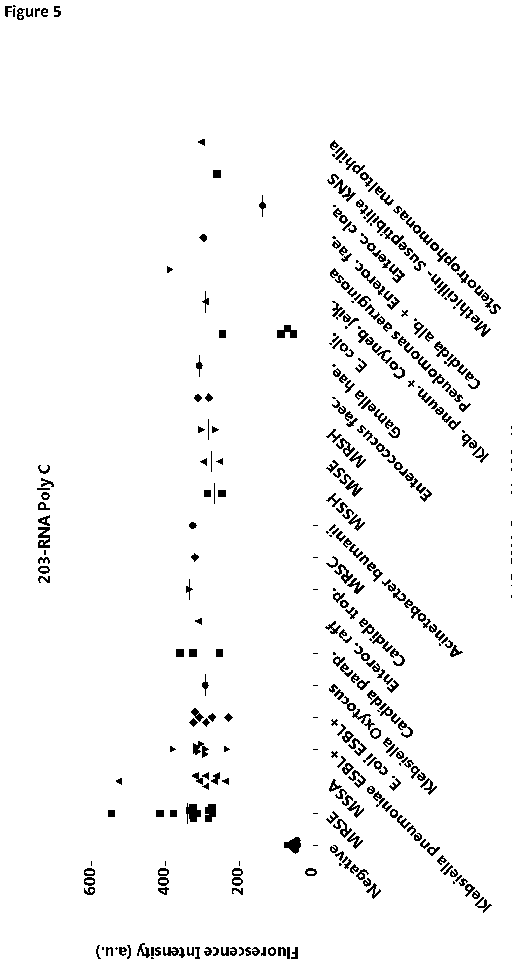

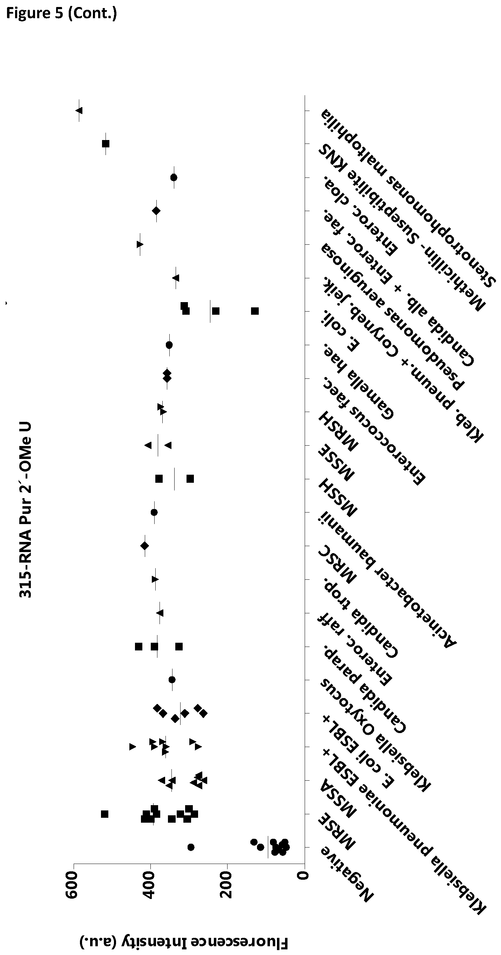

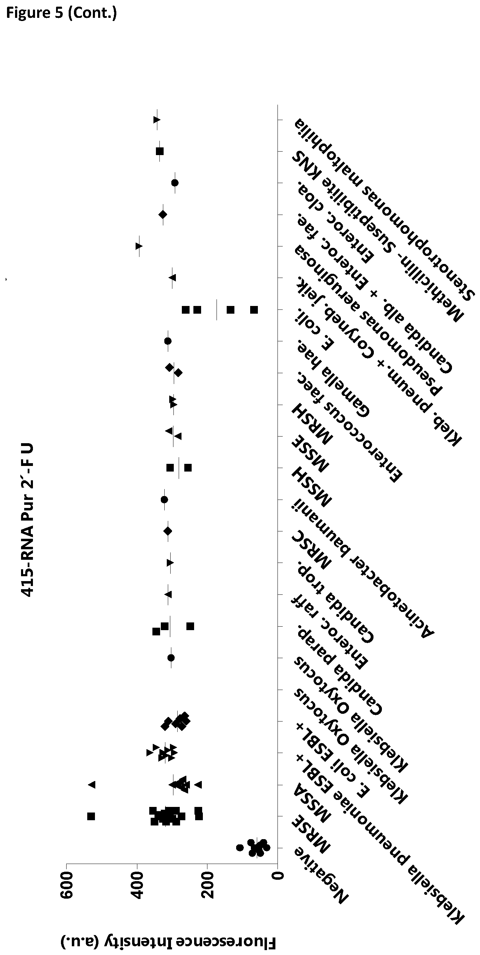

**Figure 5****:** Efficiency of the specifically identified probes SP1, SP2, SP3 (Table 2) in discriminating between healthy (n=12) and bacteria positive samples (n=70).

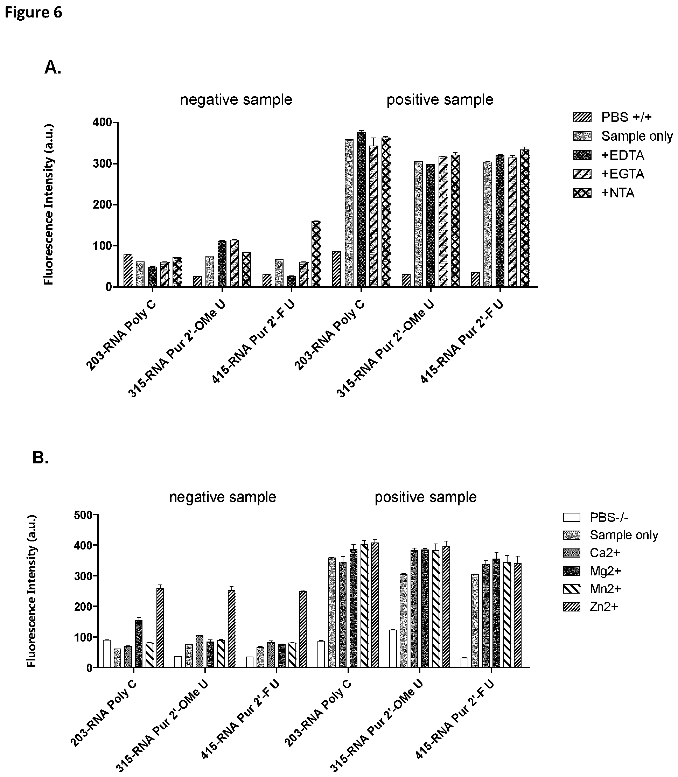

**Figure 6****:** Characterization of the nuclease activity in healthy (negative) and infected (positive) samples, using (A) chelators and (B) cations and using the 3 specific probes (SP1, SP2 and SP3) of Table 2.

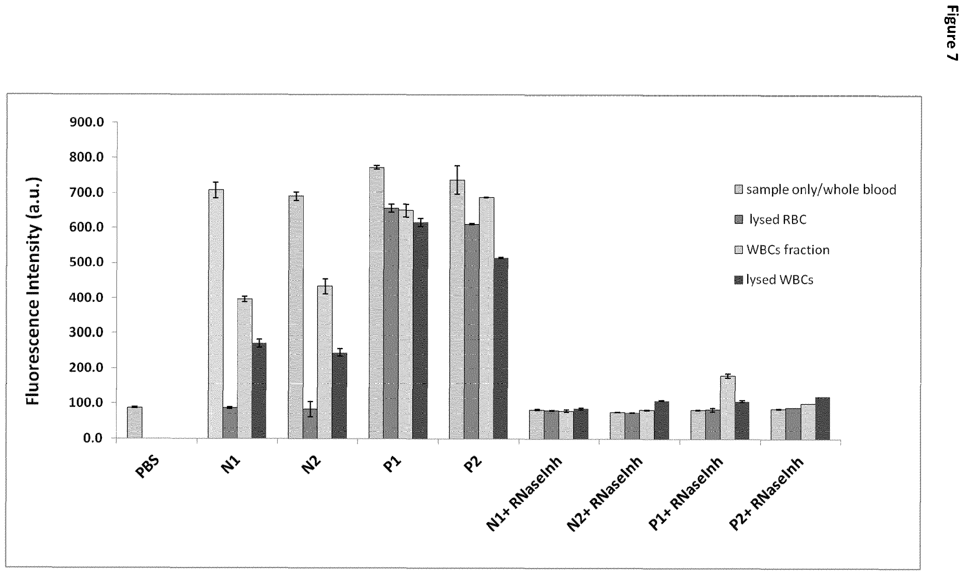

**Figure 7****:** The effect of blood cells (red blood cells, RBCs, and white blood cells, WBCs) lysis on the blood nuclease activity in samples of blood from healthy and septic patients. Nuclease activity was measured in blood samples from control, healthy individuals (N1 and N2) and patients diagnosed with sepsis (P1 and P2) using the specifically identified probe (SP1) as substrate. Nuclease activity was also measured using a commercially available ribonuclease inhibitor (RNaselnh).

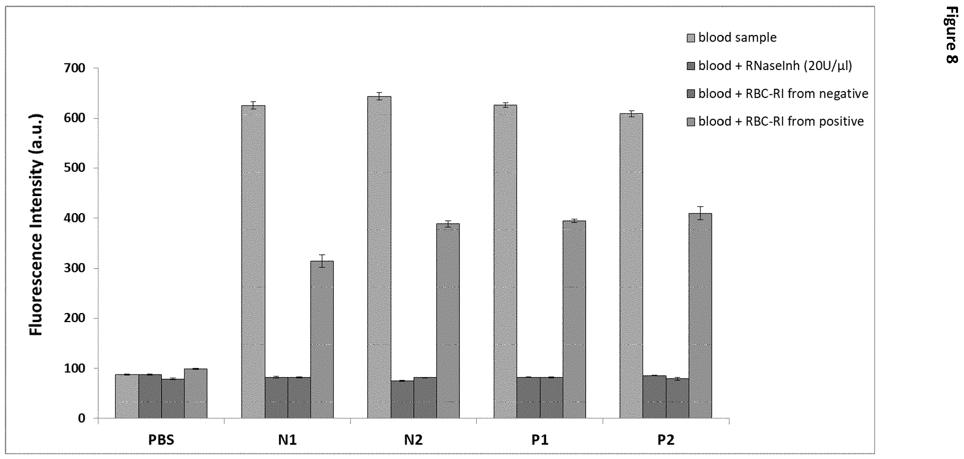

**Figure 8****: The inhibitory** effect of the commercial ribonuclease inhibitor (RNaselnh) and the RBC-derived (RI) ribonuclease inhibitors on the blood sample nuclease activity. Blood samples from healthy donors (N1 and N2) and infected blood samples from patients (P1 and P2) were tested for nuclease activity before (blood sample only, green color bar) or after inhibition with commercial RNaselnh (orange bar) or inhibitors derived from lysing the RBCs of healthy (negative) blood donor (purple bar) and infected (positive) (turquoise bar). PBS was used as control for the probe performance.

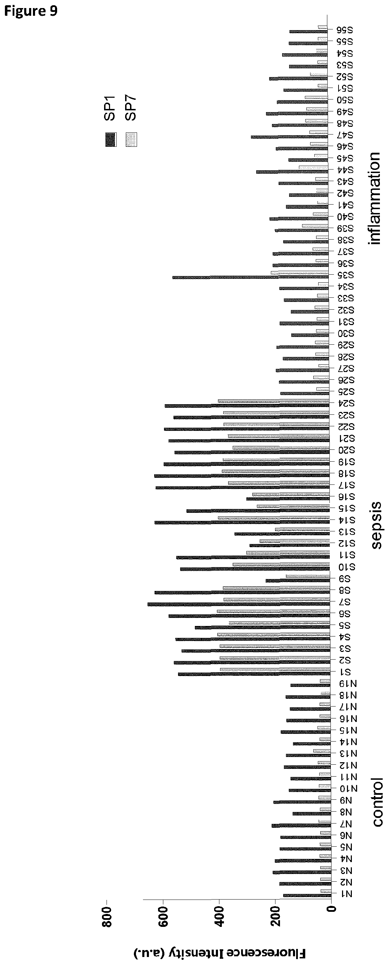

**Figure 9****:** The efficacy of SP1 and SP7 in discriminating sepsis from inflammation and control groups. Nuclease activity measurements in 24 positive samples for sepsis or septic shock (S1 to S24) and 32 negative samples for sepsis (confirmed as inflammation) (S25 to S56) along with 19 healthy controls (N1 to N19) using SP1 and SP7 nucleic acid sequences of Table 2.

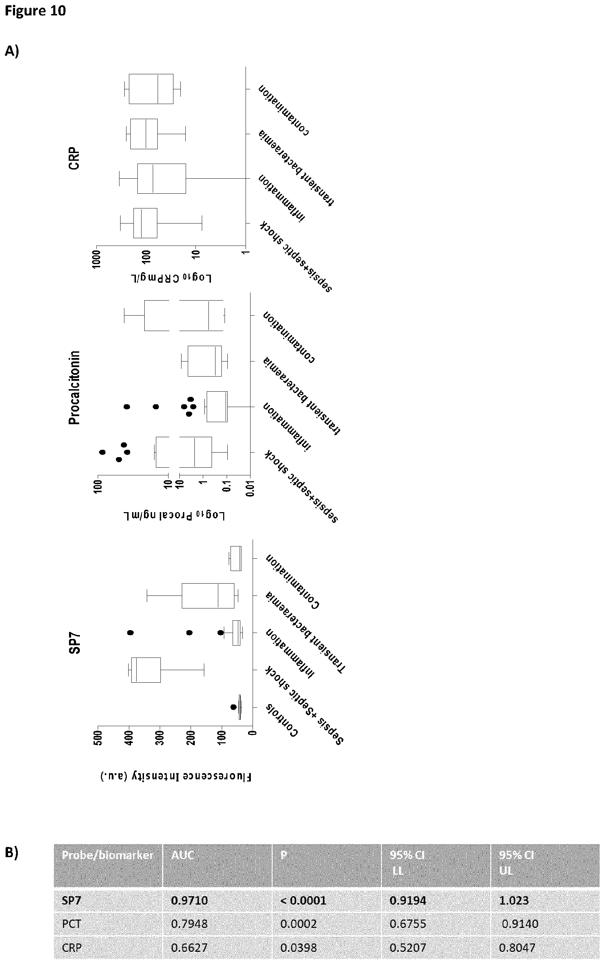

**Figure 10****:** The diagnostic accuracy of SP7 versus the clinically accepted markers of sepsis, procalcitonin (PCT) and C-reactive protein (CRP). A) Whisker plots of healthy controls group, sepsis/septic shock patient group, inflammation group, transient bacteremia group and bacteria contamination group using the specific probe SP7 ,PCT and CRP clinical values. B) Receiver operating characteristic curves (ROC) for SP7, PCT and CRP for differentiating between septic/septic shock and inflammation groups. AUC, area under the curve; CI, confidence interval (LL-lower limit, UP-upper limit), P, probability value.

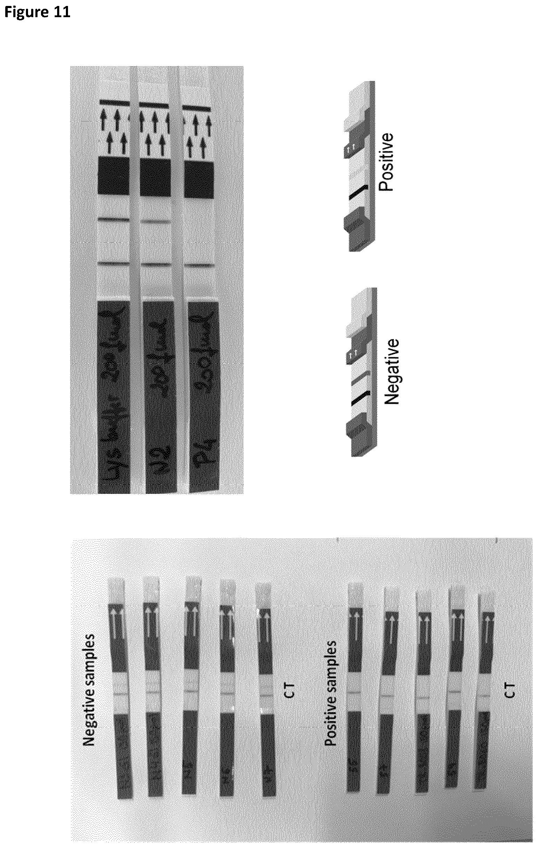

**Figure 11****:** Performance of the SP7 (150 fmoles) in the lateral flow format, with 5 negative and 5 positive clinical samples. Blue line on the strip represents control line (C); red line represents test line (T). The negative sepsis samples show two lines (blue and red), while the positive sepsis samples show only the red line. A drawing of the lateral flow system is also provided.

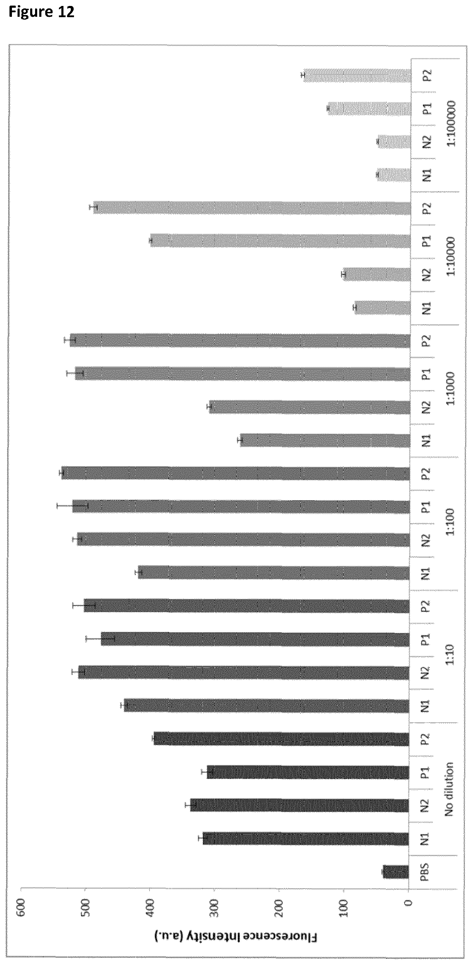

**Figure 12****:** The effect of dilution on the blood nuclease activity levels. Blood samples from healthy (negative) donors (N 1 and N2) and infected (positive) patients (P 1 and P2) were diluted in PBS buffer by a factor of 10, up to 10⁵. A dilution of 1:10⁴ was found to be an optimal dilution factor for discriminating between negative and positive blood samples.

### Detailed description of the invention

In the context of the present invention "polynucleotide" is understood as oligonucleotide substrate (single or double stranded) with the capability to recognize a specific nuclease activity derived from bacteria or mammalian cells. The polynucleotide composition consists of natural nucleic acids (DNA and/or RNA), chemically modified nucleic acids or a combination of both.

In the context of the present invention "capture tag" is understood as the modification at the end of the "polynucleotide" that allows binding with the capture molecule and/or the reporter molecule. This binding is carried out by interaction of antigen-antibody or a similar biorecognition process.

In the context of the present invention "capture molecule" is understood as recognition molecule (e.g. biotin antibody) immobilized in the detection line of the lateral flow assay, with the capability to bind one of the ends of the polynucleotide probe that was previously modified with a capture tag (e.g. biotin).

In the context of the present invention "reporter molecule" is understood as a) the molecule that provides the readout of the system and consists of a latex/gold nanoparticle, carbon particle or a similar colorimetric molecule, functionalized with a capture molecule (e.g. antibody) that recognizes the polynucleotide probe via a capture tag. b) The reporter molecule consists of a latex/gold nanoparticle that is functionalized with the polynucleotide probe, at one of its ends by coupling chemistry (e.g. amine), while the other end is modified with a capture tag (e.g. biotin).

In the context of the present invention "lateral flow assay" is understood as a simple methodology to detect, in a specific manner, the presence or absence of a target analyte in a given matrix sample. This type of assay eliminates the need for specialized and costly equipment used in conventional laboratories. Lateral flow technology is used as point of care tool (fast and in situ), in a large range of applications, from the widely used home pregnancy test to more specialized tests for clinical, food safety, environmental and industrial applications.

In the context of the present invention "a backing card" is understood as the material where all the membranes (sample, conjugate, membrane and wicking pads) are mounted and connected to each other. At the same time, the backing card provides better stability and handling to the lateral flow device.

In the context of the present invention "a sample pad" is understood as the element of the device where the sample is introduced or deposited and acts as a sponge that holds the sample fluid. Subsequently, the sample fluid is transported to the membrane by capillary action.

In the context of the present invention "the conjugate pad" is understood as the part of the device where the reporter molecules are dispensed. The reporter molecules are immediately released from the conjugate pad upon contact with the sample fluid.

In the context of the present invention "a membrane" is understood as the material (e.g. nitrocellulose) that transports the sample and provides support to the capture molecules (antibodies, aptamers etc.) and allows or enhances their binding capabilities.

In the context of the present invention "a wicking pad" is understood as element in the system that retains all the assay material acting as a waste container.

In the context of the present invention "biological sample" is understood, as used in this invention, to be any sample of, relating to, caused by, or affecting life or living organisms, biological processes, such as growth and digestion. Examples of a biological sample may include, but are not limited to cell culture, cell culture supernatant, saliva, tears, urine, blood, serum, plasma, sweat, vaginal fluids, semen, feces, mucous, breast milk, ascites, lymph, pleural effusion, synovial fluid, bone marrow, cerebro-spinal fluid, and washings from bodily cavities (e.g., bronchial lavage), hair, tissue, bones, or teeth. , the biological sample is a liquid sample. Liquid means a state of matter with definite volume but no definite shape at 25° C, like water.

### Description

As illustrated throughout the examples of the present specification, the measurement of the inhibitory activity of a subject's RBCs-RI (Red Blood Cells cytosolic ribonuclease inhibitors) on the blood/serum RNases is a novel way of differentiating healthy from septic blood and represents a valid biomarker with great clinical potential. In particular, the effect of lysing a blood biological sample, preferably the erythrocytes, and the subsequent release of the cytosolic ribonuclease inhibitors (RI), in particular the Blood ERythrocyte-derived RNase Inhibitors (BERRI), provides fora significant decrease in RNAse activity in control or uninfected samples, while little effect is observed in septic samples, showing that the reduction of nuclease activity in the control or uninfected samples is due to the specific inhibition, by the BERRI, of the blood/serum RNases, in particular of the RNAse A type endonucleases.

Thus, the present invention provides for the first time the utility and specificity of the Blood ERythrocyte-derived RNase Inhibitor Activity (BERRIA) as a biomarker for detecting sepsis in lysed blood or serum biological samples. In particular, the detection of an absence or of a significant reduction of the inhibitory activity of such RNase inhibitor over the RNase A family of endoribonucleases present in such isolated blood or serum biological sample, is indicative of sepsis in the human subject from which the biological sample was obtained. Therefore, determining or measuring the nuclease activity of the canonical RNase A family of endoribonucleases in a lysed isolated blood or serum biological sample obtained from a human subject, can serve as a biomarker of sepsis in said subject. That is, if no nuclease activity or a significant reduction of the activity is detected or measured in a blood or serum biological sample, then this is indicative that the BERRI is active and thus that the subject from which the biological sample was obtained does not suffer from sepsis. By contrast, a significant nuclease activity is detected or measured in such lysed blood or serum biological sample, then this is indicative that the BERRI is not active and thus that the subject from which the biological sample was obtained does suffer from sepsis.

In this sense, it is noted that the protein ribonuclease A (RNase A) was initially extracted from the bovine pancreas and is one of the best-characterized mammalian proteins in the literature. Over time, several other proteins with significant sequence homology were identified in mammals and other vertebrates, allowing assembly of the vertebrate-specific RNase A superfamily [Beintema, J.J.; Kleinedam, R.G. The ribonuclease A superfamily: General discussion. Cell. Mol. LifeSci. 1998, 54, 825-832. Sorrentino, S. The eight human "canonical" ribonucleases: Molecular diversity, catalytic properties, and special biological actions of the enzyme proteins. FEBS Lett. 2010, 584, 2194-2200]. The members of this superfamily can be distinguished from other exo- and endoribonucleases, which show different distributions and properties. In humans, eight secreted RNases have been described and are generally referred to as the canonical RNases (RNases 1 to 8, Int. J. Mol. Sci. 2016, 17, 1278).

These proteins show a tertiary structure that is stabilized by eight disulphide bridges, with the exception of RNase 5, which has six cysteine residues. Two histidine residues and one lysine residue determine the catalytic activity of these RNases; the lysine residue lies within the common invariant sequence motif CKxxNTF. Each RNase initially contains an N-terminal signal sequence that directs protein biosynthesis within the endoplasmic reticulum, with its final form being secretory. Moreover, the N-terminal portion of the mature extracellular RNase appears to be required for antimicrobial activity [Lander, E.S.; Linton, L.M.; Birren, B.; Nusbaum, C.; Zody, M.C.; Baldwin, J.; Devon, K.; Dewar, K.; Doyle, M.; FitzHugh, W.; et al. Initial sequencing and analysis of the human genome. Nature 2001, 409, 860-921]. This feature was demonstrated by generating N-terminus-derived peptides that showed similar antimicrobial activity.

In the present invention, we thus show that the determination of the nuclease activity of any of the eight secreted RNases (1 to 8) pertaining to the RNAse A family of endoribonucleases, generally referred to as the canonical RNases, in an isolated blood or serum sample, preferably in a lysed blood sample, serve as a biomarker of sepsis in a human subject. Hence, a first aspect of the invention refers to the *in vitro* use of the levels of the nuclease activity of the canonical RNases (RNases 1 to 8) pertaining to the RNAse A family of endoribonucleases, in an isolated blood or serum biological sample, preferably in a lysed blood sample, as a biomarker of sepsis in a human subject.

It is noted that such determination, as reflected in the above paragraph, is not limited to lysed blood or serum samples but can also be done, as reflected in figure 12, by determining the nuclease activity of any of the canonical RNases in different dilutions of the samples. It is further noted that any reference to the human RNase A type family containing the eight canonical members, shall be understood as to any of the following: human RNase 1 (hRNase1), human RNase 2 (hRNase2) also named eosinophil-derived neurotoxin (EDN), human RNase 3 (hRNase3) also named eosinophil cationic protein (ECP), human RNase 4 (hRNase4), human RNase 5 (hRNase5) also called angiogenin/ANG, human RNase 6 (hRNase6) also named RNase k6, human RNase 7 (hRNase7), and human RNase 8 (hRNase8).

In addition, a second aspect of the invention refers to the *in vitro* use of the Blood ERythrocyte-derived RNase Inhibitor Activity (BERRIA) in an isolated blood or serum biological sample as a biomarker of sepsis in a human subject, more particularly, in an isolated blood biological sample wherein the erythrocytes have been lysed thus releasing the Blood ERythrocyte-derived RNase Inhibitor.

A third aspect of the invention refers to an *in vitro* method for determining whether a subject, preferably a human subject, suffers or not from sepsis, that comprises the following steps:
a. Measuring or detecting the nuclease activity of any one or more of the eight canonical members of the human RNase A type family in an isolated blood or serum sample obtained from said subject; and
b. comparing the measurement of the nuclease activity detected in (a) to a control reference or to a reference value, or value range, or to the value, or value range, obtained from an uninfected sample,
wherein such comparison shall indicate whether the subject does or not suffer from sepsis based on the RNase activity detected in the isolated sample.

A preferred embodiment of the third aspect of the invention refers to an *in vitro* method for determining whether a subject, preferably a human subject, suffers or not from sepsis that comprises
a. Lysing the, preferably erythrocytes, of a blood, sample isolated from said subject allowing the release of the Blood ERythrocyte-derived RNase Inhibitor Activity (BERRIA);
b. Measuring or detecting the nuclease activity of the RNAse A family of endoribonucleases in said sample after the lysing of step a); and
c. comparing the measurement of the nuclease activity detected in (b) to a control reference or to a reference value, or value range, or to the value, or value range, obtained from an uninfected sample,
wherein such comparison shall indicate whether the subject does or not suffer from sepsis based on the RNAse activity detected in the isolated sample.

In a preferred embodiment of the third aspect or of any of its preferred embodiments, the reference value, or value range, used is representative of the RNAse activity in control or uninfected samples, so that if the measurement of the RNAse activity detected in the isolated sample is significantly higher than the reference value, or value range, or the value obtained from an uninfected sample, is indicative that the subject suffers from sepsis, otherwise this is indicative that the subject does not suffer from sepsis.

In another preferred embodiment, a reference value, or value range, is used in the method of the third aspect of the invention which is representative of the RNAse activity in samples with sepsis so that if the measurement of the RNAse activity detected in the isolated biological sample being tested is significantly lower than the reference value, or the value range, then this is indicative that the subject does not suffer from sepsis, otherwise this is indicative that the subject does suffer from sepsis.

In yet another preferred embodiment, the sepsis is due or caused by a microbial, bacterial, viral or fungi infection.

It is noted that the measurement or detection of the nuclease activity of any of the previous aspects of the present invention can be performed by a large variety of techniques. In this sense, it is known that RNase A family members bind and catalyze the phosphodiester bond cleavage in a wide variety of nucleotide substrates of different lengths and sequences. Past studies have suggested substrate preferences at the pyrimidine binding site for some of the human RNases: hRNase1 showed a preference for cytosine (C) over uridine (U), while hRNases 2, 3, 4 and 6 showed a preference for U over C [Sorrentino S. The eight human "canonical" ribonucleases: Molecular diversity, catalytic properties, and special biological actions of the enzyme proteins. FEBS Letters. 2010; 584(11):2194-200. https://doi.org/10.1016/j.febslet.2010.04.018 PMID: 20388512. Prats-Ejarque G, Arranz-Trullen J, Blanco JA, Pulido D, Nogues MV, Moussaoui M, et al. The first crystal structure of human RNase 6 reveals a novel substrate-binding and cleavage site arrangement. Biochemical Journal. 2016; 473(11):1523-36]. In contrast, the purine binding site was shown to prefer adenosine (A) in these hRNases [1,2,3].

In order to measure the nuclease activity, we have identified a number of oligonucleotide substrates (Table 2) as specific substrates for the blood RNases, particularly RNase A family of riboendonucleases, that are able to discriminate between an infected blood (septic) sample and a healthy blood sample. These oligonucleotide substrates share the following features in common: i) short length, ii) RNA pyrimidines as preferred substrate for sepsis-derived nucleases, iii) chimeric sequences, containing nucleoside analogues modified and natural RNA pyrimidines nucleotides (unmodified). A cleavage region between 1 to 3 RNA pyrimidines flanked by chemically modified nucleotides increase the specificity for RNases derived from sepsis. It is particularly noted that the cleavage motif -CUC - in (SP7 of table 2) flanked by chemically modified nucleotides has shown increased specificity and sensitivity for detecting sepsis-derived RNases.

Therefore, to measure the nuclease activity according to any of the previous aspects, oligonucleotide substrates (from hereinafter oligonucleotides of the invention) can be used that comprise i) 5- 30 nucleotides in length, and ii) a cleavage region between 1 to 3 RNA pyrimidines, preferably flanked by chemically modified nucleotides, wherein such cleavage region is susceptible of being cleaved by any of the eight canonical secreted RNases (1 to 8) pertaining to the RNase A family. Preferably, said cleavage region is selected from any of those identified in bold in table 2. More preferably, the cleavage motif is -CUC -, as identified in SP7 of table 2, preferably flanked by chemically modified nucleotides that has shown increased specificity and sensitivity for detecting sepsis-derived RNases. Still more preferably, said oligonucleotide substrates are selected from any of the list identified in Table 2.

In addition, in a preferred embodiment of the third aspect of the invention, the method of the third aspect of the invention comprises measuring or detecting a fluorescence signal of the sample after contacting said biological sample with at least one probe comprising an oligonucleotide of the invention, a fluorophore operably linked to the oligonucleotide, and a quencher operably linked to the oligonucleotide, wherein the oligonucleotide is capable of being cleaved by the RNAse A family of endoribonucleases in said sample, preferably after the blood lysis, more preferably after erythrocyte lysing. It is noted that such oligonucleotides of the invention useful to practice this specific embodiment of the invention are described in WO2013033436, which is herein fully incorporated by reference. Preferably, such oligonucleotides of the invention, are capable of being cleaved by the RNAse A family of endoribonucleases and comprise chemically modified RNA flanked with a fluorophore on one end and a fluorescence quencher on the other end. Upon cleavage of the probes by the RNAse A family of endoribonucleases, the fluorophore diffuses away from the quencher and exhibits fluorescence.

Thus, in one preferred embodiment, the method of the third aspect of the invention can be thus implemented by detecting a fluorescence signal, such method would preferably comprise: 1) incubating an oligonucleotide substrate of the invention in the sample, for a time sufficient for cleavage of the Substrates(s) by the RNAse A family of endoribonucleases in the, preferably lysed, blood or the serum sample obtained from the said blood sample, wherein the Substrate(s) comprises a single-stranded nucleic acid molecule containing at least one RNA pyrimidine residue at an internal position that functions as a nuclease (e.g., ribonuclease) cleavage site, a fluorescence reporter group on one side of the cleavage sites, and a fluorescence-quenching group on the other side of the cleavage site, and 2) detection of a fluorescence signal, wherein detection of a fluorescence signal indicates that the RNAse A family of endoribonucleases cleavage event has occurred, and, therefore, the sample contains said nuclease (e.g., ribonuclease) activity which is indicative that the subject does not suffer of sepsis. The oligonucleotide substrates of the invention are compatible with different detection modalities (e.g., fluorometry). For this specific embodiment, it is mandatory that the Substrate oligonucleotides of the invention comprise a fluorescent reporter group and a quencher group in such physical proximity that the fluorescence signal from the reporter group is suppressed by the quencher group. Cleavage of the Substrate with a nuclease (e.g., ribonuclease) enzyme leads to strand cleavage and physical separation of the reporter group from the quencher group. Separation of reporter and quencher eliminates quenching, resulting in an increase in fluorescence emission from the reporter group. When the quencher is a so-called "dark quencher", the resulting fluorescence signal can be detected by direct visual inspection (provided the emitted light includes visible wavelengths).

On the other hand, to enable visual detection of the method of the third aspect of the invention implemented by detecting a fluorescence signal, the quenching group is itself not capable of fluorescence emission, being a "dark quencher". Use of a "dark quencher" eliminates the background fluorescence of the intact Substrate that would otherwise occur as a result of energy transfer from the reporter fluorophore. In one embodiment, the fluorescence quencher comprises dabcyl (4-(4'-dimethylaminophenylazo)benzoic acid). In one embodiment, the fluorescence quencher is comprised of QSY^{™}-7 carboxylic acid, succinimidyl ester (N,N'- dimethyl-N,N'-diphenyl-4-((5-t-butoxycarbonylaminopentyl)aminocarbonyl)piperidinylsulfonerhodamine; a diarylrhodamine derivative from Molecular Probes, Eugene, Oreg.). Any suitable fluorophore may be used as reporter provided its spectral properties are favorable for use with the chosen quencher. A variety of fluorophores can be used as reporters, including but not limited to, fluorescein, tetrachlorofluorescein, hexachlorofluorescein, rhodamine, tetramethylrhodamine, Cy-dyes, Texas Red, Bodipy dyes, and Alexa dyes.

The method of the third aspect of the invention implemented by detecting a fluorescence signal may thus preferably proceed in two steps. First, the, preferably lysed, test biological sample is mixed with the Substrate oligonucleotide of the invention and incubated. Substrate can be mixed alone with the test sample or will be mixed with an appropriate buffer. Second, detection of fluorescence is performed. As fluorescence above background indicates fluorescence emission of the reaction product, i.e. the cleaved Substrate, detection of such fluorescence indicates that RNase activity is present in the test sample. The method provides that this step can be done with unassisted visual inspection. In particular, visual detection can be performed using a standard ultraviolet (UV) light source of the kind found in most molecular biology laboratories to provide fluorescence excitation. Substrates of the invention can also be utilized in assay formats in which detection of Substrate cleavage is done using a multi-well fluorescence plate reader or a tube fluorometer.

On a still further note, as previously indicated, the detection or measurement of the nuclease activity of any of the aspects of the present invention can be performed by a variety of techniques, wherein for example a further technique is by detecting or smeasuring the nuclease activity, preferably visually, by using a lateral flow device. It is noted that such lateral flow devices useful to practice the present invention, in particular the method of the third aspect of the invention, are thoroughly described in WO2019/043187, which is herein wholly incorporated by reference.

Thus the present invention further includes lateral flow devices and kits containing the same for implementing any of the aspects herein indicated, in particular the method of the third aspect of the invention. Such kits may optionally contain one or more of: a positive control nuclease, a probe substrate, DNase/RNase-free water, a buffer, and other reagents.

Lateral Flow tests, also known as Lateral Flow Immunochromatography (LFIC) tests or just Lateral Flow Immunoassays (LFI), are simple small devices intended to detect a target analyte in a given sample. These tests are simple, fast, and cheap and do not require specialized and costly equipment nor qualified personnel. Even though the LFIC technology was born in the early 80s, it is still going to be a major player in the next decades in the rapid test industry. In the beginning, LFIC tests were exclusively used in medical diagnostics (either for home testing, point of care testing, or laboratory use) but their presence in other areas are now very common. The first application was the well-known home pregnancy test. Although there are several commercially available semi-quantitative tests, LFIC tests are mainly qualitative tests and they just give a positive or negative result based on the presence or absence of the target analyte at a specific concentration.

The technology is based on a series of capillary beds with porous materials that has the capacity to transport liquids spontaneously. When the target analyte is big (e.g. a protein) a sandwich format is commonly used. Usually, the first element (the conjugate pad) has stored the so-called colloids, a dried format of bioactive particles (mainly gold nanoparticles or latex microparticles bound to a specific antibody against the target analyte) in a salt-sugar matrix. This conjugate pad usually acts also as the sample pad and it contains everything to facilitate the recognition of the target antigen by the colloid. Once the conjugate pad is soaked by the sample, the fluid dissolves the salt-sugar matrix and the colloids. Therefore, the sample and the colloid mix while they flow through the porous structure. In this way, the analyte binds to the colloid and the complex is transported into the nitrocellulose membrane. This material has one specific area called test line where a third molecule (usually another different antibody against the target analyte) has been immobilized. When the complex (colloid-antigen) reaches the test line it gets bound to this antibody and after a while, when more and more fluid has flown, colloids accumulate, and the test line acquires colour. Typically, there is also a control line (after the test line) that captures the colloids that have not bound to the test line. In the control line, a fourth molecule (could be an antibody against the Fc fraction of the first antibody) is adsorbed and its color acquisition ensures the test functionality. After passing these reaction zones the fluid enters the final porous material, the wick or sink that simply acts as a waste container.

In this sense, we have developed specific LFPC strips that can detect non-degraded polynucleotide probes in a given sample. Accordingly, the presence of a polynucleotide probe will give rise to a coloured test line, which according to the methodology of the third aspect of the invention indicates that the subject does not suffer from sepsis. On the other hand, if a specific nuclease is present in the, preferably lysed, sample it will eventually produce a complete degradation of the polynucleotide probe, and consequently, no test line will be seen, which according to the methodology of the third aspect of the invention indicates that the subject does suffer from sepsis, preferably since if a lysed sample is used, no BERRI activity would be detected in the sample. Such system shall provide for a rapid and sensitive molecular approach useful for implementing the method of the third aspect of the invention. For that purpose, the present invention thus provides for an in vitro use of a polynucleotide sequence of the invention susceptible of being cleaved by the RNAse A family of endoribonucleases present in a, preferably lysed, blood or the serum obtained from that said blood biological sample, in a lateral flow assay.

To practice the present invention, a large variety of different lateral flow system devices could be used, however, two devices to which the present invention is not limited to are herein provided just for illustrative purposes:
A first lateral flow device comprising a support suitable for lateral flow, which in turn comprises:
a1. A backing card; and
a2. A sample pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for capturing the capture molecule of the polynucleotide sequence, and wherein the wicking pad is located at the end of the membrane.

Preferably, said first lateral flow device is comprised in a kit which further comprises a probe comprising an oligonucleotide sequence of the invention susceptible of being cleaved by the RNAse A family of endoribonucleases, wherein the oligonucleotide sequence is characterized by comprising a capture tag and a reporter molecule at each end of the sequence, for determining whether a subject suffers or not from sepsis.

A second lateral flow device comprising a support suitable for lateral flow, which in turn comprises:
a1. A backing card; and
a2. A sample pad, a membrane and a wicking pad, all of them on top of the backing pad, wherein the membrane is located after the sample pad so that the sample flows from the sample pad to the membrane, wherein the membrane comprises a test line which in turn comprises molecules immobilized in the test line for capturing the capture molecule of the polynucleotide sequence; and wherein the support further comprises a conjugate pad between the sample pad and the membrane, which in turn comprises a reporter molecule.

Preferably, said second lateral flow device is comprised in a kit which further comprises a probe comprising an oligonucleotide sequence of the invention susceptible of being cleaved by the RNAse A family of endoribonucleases, wherein the oligonucleotide sequence is characterized by comprising two capture tags at each end of the sequence, wherein each capture tag is different from the other and wherein one of the capture tags is capable of binding a reporter molecule, for determining whether a subject suffers or not from sepsis.

In certain embodiments, the said polynucleotides sequences of the invention susceptible of being cleaved by the RNAse A family of endoribonucleases, are short oligonucleotide probes (substrate) composed of nucleic acids and flanked with a capture tag on one end and i) a reporter molecule or ii) a further capture tag capable of binding the reporter molecule located in the conjugate pad of a support of a lateral flow device, on the other end. Upon cleavage of the probes by the RNAse A family of endoribonucleases in the, preferably lysed, blood or the serum obtained from that said blood sample, the capture tag is released away from the reporter molecule indicating the presence of said nucleases in a biological sample in a lateral flow device. Cleavage of the oligonucleotide by the RNAse A family of endoribonuclease leads to strand cleavage and physical separation of the capture group from the reporter group. Separation of reporter and capture eliminates the possibility of detecting a signal in the test line of the lateral flow device thus resulting in the detection of nuclease activity in the sample. The absence of the resulting signal can be detected by direct visual inspection of the lateral flow device.

In addition, in a further specific embodiment of the present invention, the implementation of method of the third aspect of the invention, comprises: 1) incubating a synthetic Substrate or mixture of oligonucleotide Substrates of the invention in the, preferably lysed, sample, for a time sufficient for cleavage of the Substrate(s) by the RNAse A family of endoribonucleases, wherein the Substrate(s) comprises a single-stranded (or double-stranded) nucleic acid molecule containing at least one RNA pyrimidines residue or chemically modified RNA pyrimidines residue at an internal position that functions as a nuclease (RNAse A family of endoribonuclease) cleavage site, a capture tag on one end and i) a reporter molecule or ii) a further capture tag capable of binding the reporter molecule located in the conjugate pad of a support of a lateral flow device, on the other end, and 2) detecting the result, preferably visually, in particular whether the substrate has been cleaved or not by a test sample, by using a lateral flow device.

Yet another preferred embodiment of the present invention, relates to the in vitro use of a kit or a lateral flow device which comprises:
(a) a probe comprising an oligonucleotide sequence of the invention susceptible of being cleaved by the RNAse A family of endoribonucleases, wherein the oligonucleotide sequence is characterized by comprising a capture tag and a reporter molecule at each end of the sequence, for determining whether a subject suffers or not from sepsis.
(b) a support suitable for lateral flow that comprises:
   b1. a backing card; and
   b2. a sample pad, a membrane and a wicking pad, all of them on top of the backing pad, wherein the membrane is located after the sample pad so that the sample flows from the sample pad to the membrane, wherein the membrane comprises at least two test lines which in turn comprises molecules immobilized in the test line for capturing the capture molecule of the oligonucleotide sequence and wherein the wicking pad is located at the end of the membrane;
for implementing the methodology of the third aspect of the invention.

Preferably, said said lateral flow device comprises:
(a) the probe with any of SEQ ID NO.: 1-3, or 7 characterized by comprising a capture tag and a reporter molecule at each end of the sequence, for determining whether a subject suffers or not from sepsis.
(b) a support suitable for lateral flow that comprises:
   b1. a backing card; and
   b2. a sample pad, a membrane and a wicking pad, all of them on top of the backing pad, wherein the membrane is located after the sample pad so that the sample flows from the sample pad to the membrane, wherein the membrane comprises at least a test line which in turn comprises molecules immobilized in the test line for capturing the capture molecule of the SEQ ID NO.: 7 and wherein the wicking pad is located at the end of the membrane.

Also preferably, said lateral flow device comprises:
(a) a probe comprising an oligonucleotide sequence of the invention susceptible of being cleaved by the RNAse A family of endoribonucleases, wherein the oligonucleotide sequence is characterized by comprising two capture tags at each end of the sequence, wherein each capture tag is different from the other and wherein one of the capture tags is capable of binding a reporter molecule, for determining whether a subject suffers or not from sepsis.
(b) a support suitable for lateral flow that comprises:
   b1. a backing card; and
   b2. a sample pad, a membrane and a wicking pad, all of them on top of the backing pad, wherein the membrane is located after the sample pad so that the sample flows from the sample pad to the membrane, wherein the membrane comprises at least two test lines which in turn respectively comprises molecules immobilized in each test line for capturing the capture tag of the oligonucleotide sequence, wherein the wicking pad is located at the end of the membrane,
and wherein the system further comprises a conjugate pad between the sample pad and the membrane, which in turn comprises a reporter molecule.

Also preferably, said lateral flow device comprises:
(a) the probe with any of SEQ ID NO.: 1-3, or 7 characterized by comprising two capture tags at each end of the sequence, wherein each capture tag is different from the other and wherein one of the capture tags is capable of binding a reporter molecule, for determining whether a subject suffers or not from sepsis.
(b) a support suitable for lateral flow that comprises:
   b1. a backing card; and
   b2. a sample pad, a membrane and a wicking pad, all of them on top of the backing pad, wherein the membrane is located after the sample pad so that the sample flows from the sample pad to the membrane, wherein the membrane comprises at least two test lines which in turn respectively comprises molecules immobilized in each test line for capturing the capture tag of the probe with SEQ ID NO.: 7, wherein the wicking pad is located at the end of the membrane,
and wherein the system further comprises a conjugate pad between the sample pad and the membrane, which in turn comprises a reporter molecule.

It is further noted that the lateral flow device or kit described in the third aspect of the invention may further comprise a control line.

Finally it is noted that the detection of the activity of the RNAse A family of endoribonucleases of the aspects of the invention can be performed by further techniques such as nuclear magnetic resonance (NMR).

The following examples merely serve to illustrate the present invention but do not limit the same.

### Examples

### Example 1: First screening of blood/serum samples using 12 probes library

The nuclease activity profile of paired blood and serum samples infected with various pathogens was screened with a library of 12 probes, either naked (DNA or RNA) or containing chemical modifications at the 2'position of at the sugar ribose (2'- O methyl and 2'-fluoro). The nuclease activity of infected blood with *Methicillin resistant staphylococcus epidermidis* (MRSE), *Methicillin sensitive staphylococcus aureus* (MSSA) and *Klebsiella oxytoca* and PBS was measured by fluorescence intensity (arbitrary units).

The results are shown in Figure 1. High nuclease activity was detected in both types of samples, blood and serum, using the probes. However, no significant differences in the nuclease activity profile between the 3 different pathogens were observed.

### Example 2: Second screening of blood/serum samples using 80 probes library before or after cell lysis

A second round of screening was conducted in serum samples infected with MRSE or MSSA, similarly as described in example 1, but using an extended library containing the 80 nucleic acid probe sequences shown in Table 1 and a healthy blood sample (N1) as control sample. The results are shown in Figure 2.

Further, in this Example, healthy (N1-3) or pathogen infected blood samples (infected with *MRSE, Klebsiella, E .coli-1, E. coli-2* or *MSSA)* were screened for nuclease activity, before or after blood lysis using the extended library containing the 80 nucleic acid probe sequences of Table 1.

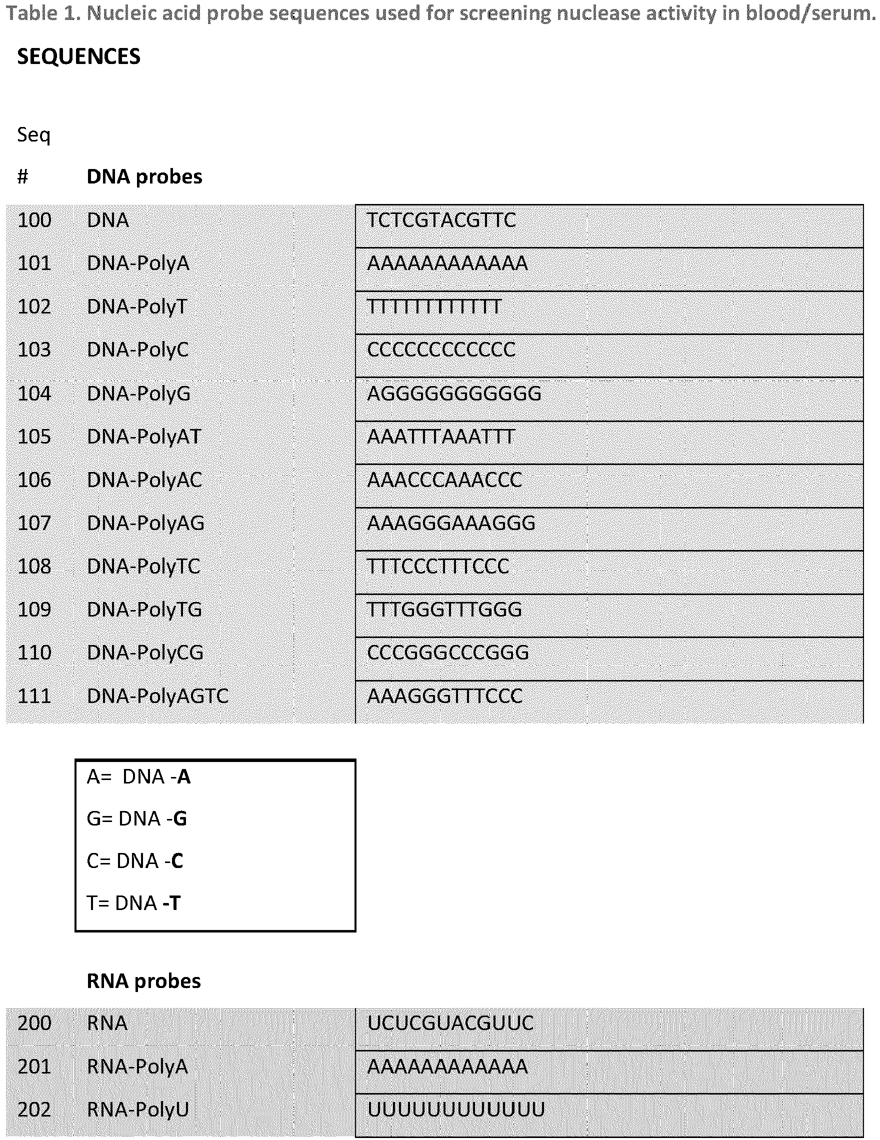

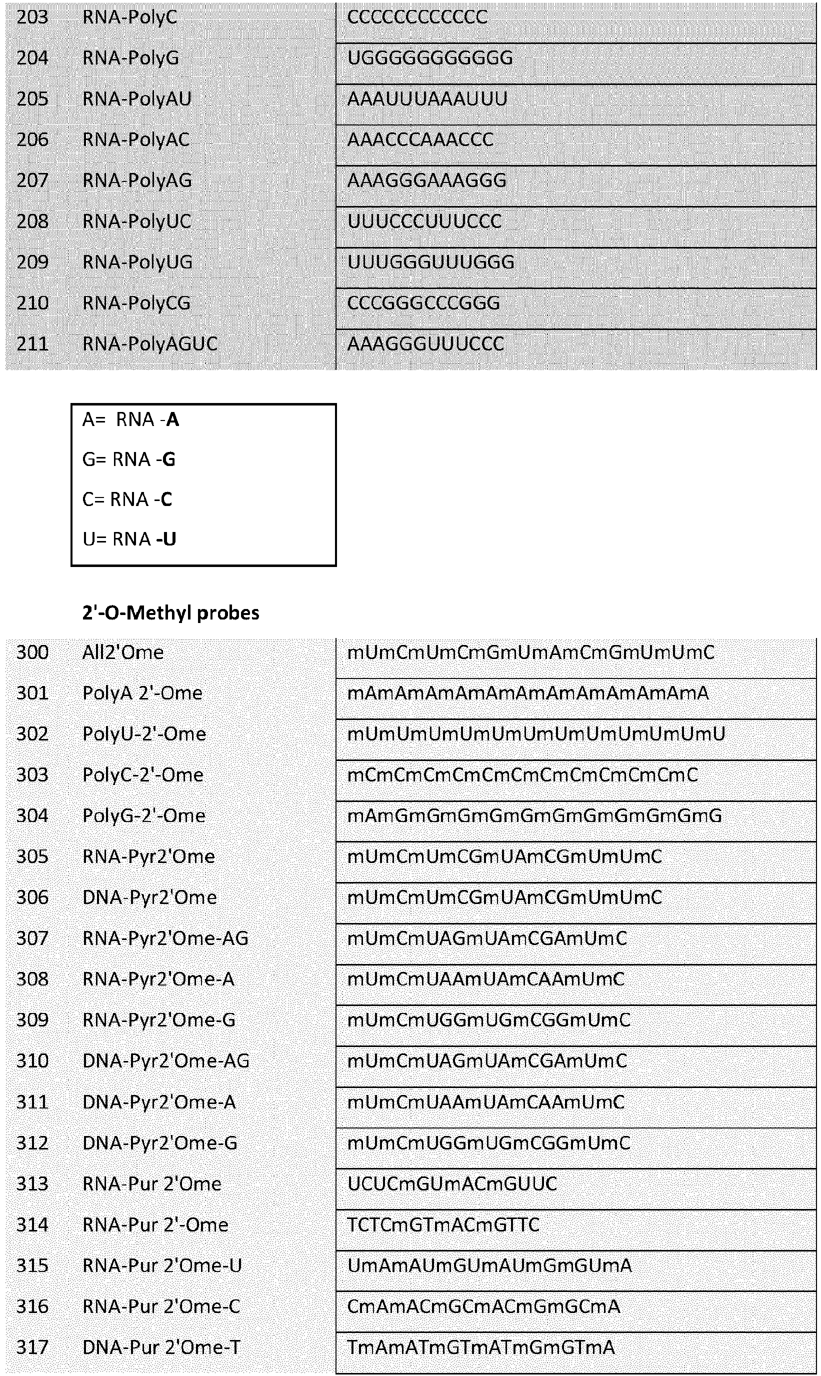

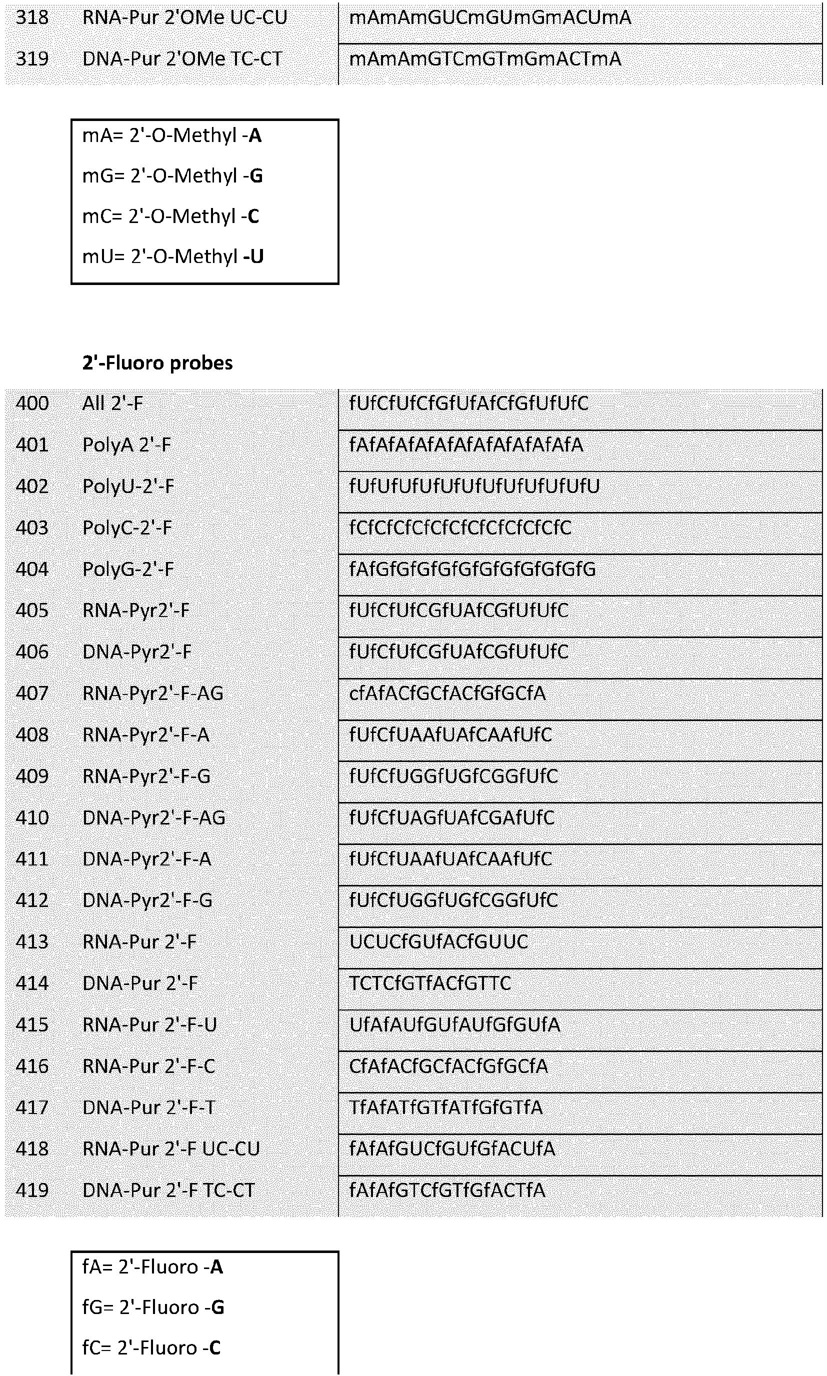

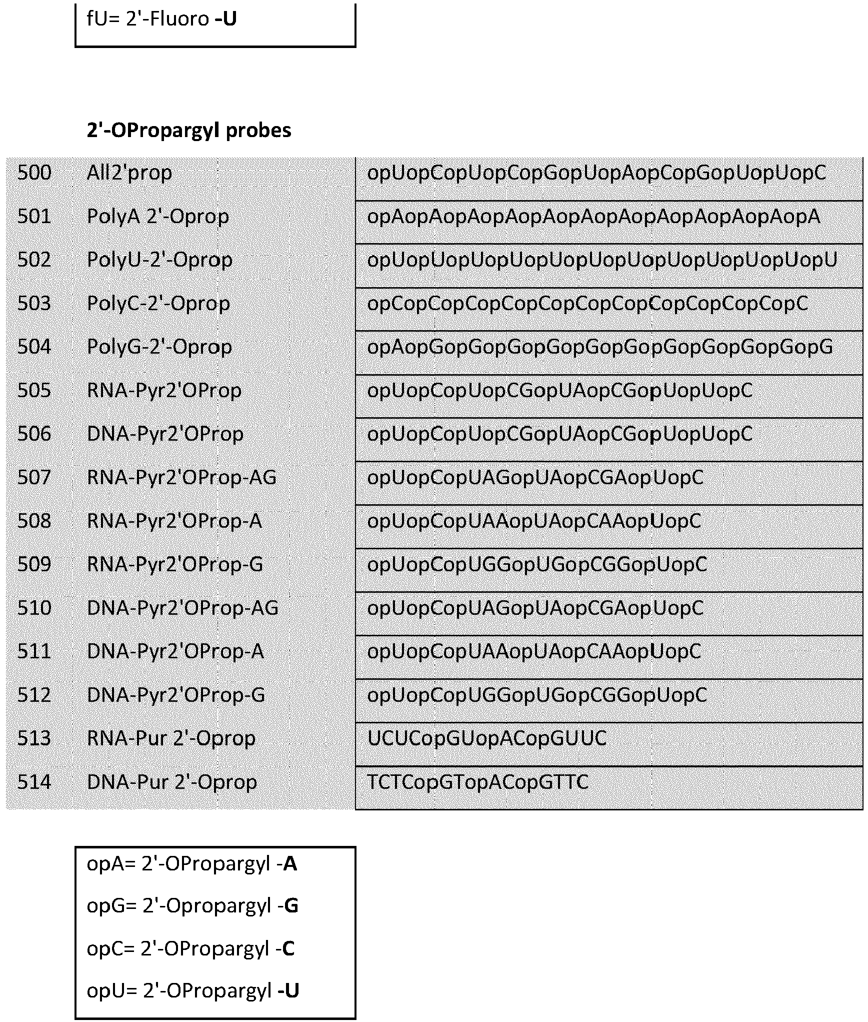

The results are shown in Figure 3, where Figure 3A shows the nuclease activity measured before blood lysis, and Figure 3B shows the nuclease activity measured after blood lysis. High ribonuclease type of activity was seen in both, before and after blood lysis. Importantly, by lysing the blood cell components, a sharp and significant decrease in nuclease activity of the healthy samples (N1-4, blue bars Figure 3B) was observed, allowing for a clear discrimination between the negative and positive sepsis blood samples (Figure 3B).

Lastly, Figure 4 shows a detailed probe performance for discriminating between healthy (negative) and infected serum samples, where the fluorescence intensity values correspond to the degradation efficiency of the nucleic acid probe substrate by the sample nucleases.

### Example 3: Degradation efficiency by nucleases present in a larger number of infected blood samples

In view of the results of the screening of the previous example, 12 specific probes that resulted in better discrimination between the negative and positive groups were selected for further experiments. These probes are shown in Table 2 (SP1-SP12). Next, a larger number of blood samples that tested positive (microbiologically) for a variety of bacteria (n=70) along with normal, healthy controls (n=11) were tested using the 3 first identified probes from Table 2 (SP1-3).

**Table 2. Specific nucleic acid probe names and sequences (SEQ ID NO 1 to 12)**

| **Specific probe number** | **Specific probe name** | **Sequence** |
|---|---|---|
| SP1 | 203-RNA PolyC | CCCCCCCCCCCC (RNA) |
| SP2 | 315-RNA-Pur2'-OMe U | UmAmAUmGUmAUmGmGUmA (RNA and 2'-O-Methyl) |
| SP3 | 415-RNA-Pur 2'-F U | UfAfAUfGUfAUfGfGUfA (RNA and 2'-Fluoro) |
| SP4 | -C- | mUmUmUmU**C**mUmUmUmU (RNA and 2'-O-Methyl) |
| SP5 | -CC- | mUmUmUmU**CC**mUmUmUmU (RNA and 2'-O-Methyl) |
| SP6 | -CCC- | mUmUmUmU**CCC**mUmUmUmU (RNA and 2'-O-Methyl) |
| SP7 | -CUC- | mUmUmUmU**CUC**mUmUmUmU (RNA and 2'-O-Methyl) |
| SP8 | -UC- | mUmUmUmU**UC**mUmUmUmU (RNA and 2'-O-Methyl) |
| SP9 | -CU- | mUmUmUmU**CU**mUmUmUmU (RNA and 2'-O-Methyl) |
| SP10 | -U- | mUmUmUmU**U**mUmUmUmU (RNA and 2'-O-Methyl) |
| SP11 | -UU- | mUmUmUmU**UU**mUmUmUmU (RNA and 2'-O-Methyl) |
| SP12 | -UUU- | mUmUmUmU**UUU**mUmUmUmU (RNA and 2'-O-Methyl) |

Figure 5 illustrates the efficient degradation of the three probes (SP1-SP3) by nucleases present in the infected blood samples (given by the high fluorescence signals), while all negative samples cluster showed very low intensity levels, meaning very little degradation occurred. Notably, the probes were cleaved with similar efficiency by all samples tested, irrespective of the type of bacteria, showing that this effect is caused due to a host response scenario.

### Example 4: The nuclease activity measured is from a member of RNAse A family of endoribonucleases

To further characterize the type of nucleases whose activity was being measured, the 3 specific probes (SP1, SP2 and Sp3) used in the previous example were used with various chelators and cations and in negative and positive-infected lysed blood samples. The results are shown in Figure 6A (chelators) and B (cations).

As it can be seen in Figure 6, the ribonuclease identified in the lysed blood samples is very stable and does not require divalent cations for its activity, indicating that it belongs to the RNAse A family of endoribonucleases. This family of endoribonucleases are secreted by a range of tissues and immune cells and their general role is to eliminate cellular self-RNA and/or pathogenic non-self RNA species in the extracellular space to prevent infection by pathogens and autoimmune responses.

### Example 5: Nuclease activity detection in patients with sepsis and nuclease activity inhibition by RNaselnh

To further measure the nuclease activity in blood samples from control, healthy individuals (N1 and N2) and patients diagnosed with sepsis (P1 and P2), an assay using the specifically identified probe (SP1) (Table 2) was performed. In this case, both red blood cells (RBCs) and white blood cells (WBCs) were lysed for nuclease activity measurement. The results are shown in Figure 7.

Figure 7 shows the effect of cell lysing and subsequent release of the cytosolic ribonuclease inhibitors (RI) on the blood nuclease activity. As it is shown, at baseline, the whole blood RNAse activity is high (nude bar). However, after RBC lysing (darker orange bars), a significant decrease in RNAse activity is seen in the control samples N1 and N2, while little effect is observed in the septic samples P1 and P2, showing that the reduction of nuclease activity seen in control samples is due to the specific inhibition of the RNAse A type nuclease in blood.

To confirm that the reduction of nuclease activity is due to the specific inhibition of the RNAse A type nucleases in blood, a commercially available ribonuclease inhibitor (RNaselnh) was also used in the sample. As it can be appreciated from Fig 7, RNaselnh efficiently reduced the nuclease activity of all samples. Moreover, the WBCs faction was evaluated, and the effect was found to be somewhat moderate, with respect to the cytosolic RI.

### Example 6: RBCs lysates as source of ribonuclease inhibitors (RI).

In this Example, RBCs lysates from the negative samples (N1, N2, N3 and N4) or positive samples (P3 and P4) were used as source of RI and were added to the whole blood samples, along with the commercial RNaselnh. The results are shown in Figure 8. As it can be seen, the RBC-derived ribonuclease inhibitors (from control samples) and the commercial RNaselnh were equally effective in inhibited the nuclease activity (Figures 8 A and B). On the other hand, the RBC-RI derived from the positive samples (Fig 8B) did not have any effect on the whole sample nuclease activity, showing that the septic erythrocytes do not have active RI.

### Example 7: Probes' ability to differentiate inflammation from sepsis

The examples above show that the measurement of inhibitory activity of RBCs-RI on the blood RNases is a novel way of differentiating healthy from septic blood and represents a valid biomarker with great clinical potential.

The utility and specificity of the Blood ERythrocyte-derived RNase Inhibitor Activity (BERRIA) biomarker for detecting sepsis was further tested in a cohort of patient samples: 24 positive samples for sepsis or septic shock (S1 to S24) and 32 negative samples for sepsis (confirmed as inflammation) (S25 to S56) along with 19 healthy controls (N1 to N19) using the nucleic acid sequences SP1 and SP7 in Table 2.

The results are shown in Figure 9, where it can be seen that the specific nucleotides are able to differentiate sepsis infection from inflammation and control groups.

### Example 8: Probes' accuracy in comparison with other markers for sepsis

Lastly, the diagnostic accuracy of SP7 was compared with the clinically accepted markers of sepsis: Procalcitonin and CRP. The results are shown in Figure 10 A and B and confirm the potential of these probes for detecting sepsis with high specificity and accuracy.

### Example 9: Detection of sepsis by using a lateral flow system

Once SP7 was successfully tested in clinical samples in the fluorescence format, the probe was next synthesized for the lateral flow optimization.

Figure 11 shows the performance of the SP7 (150 fmoles) in the lateral flow format, with 5 negative and 5 positive clinical samples. Blue line on the strip represents control line (C); red line represents test line (T). The negative sepsis samples show two lines (blue and red), while the positive sepsis samples show only the red as showing below.

## Claims

1. An *in vitro* use of the levels of the nuclease activity of the canonical RNases pertaining to the RNAse A family of endoribonucleases, in an isolated blood or serum biological sample taken from a human subject, as a biomarker of sepsis in said subject.

2. The in vitro use according to claim 1, wherein the levels of the nuclease activity of the canonical RNases are determined in an isolated blood biological sample after lysing said sample.

3. An *in vitro* use of the Blood ERythrocyte-derived RNase Inhibitor Activity (BERRIA) in an isolated lysed blood biological sample taken from a human subject as a biomarker of sepsis in said subject.

4. An *in vitro* use of the levels of the nuclease activity of the RNAse A family of endoribonucleases in an isolated blood or serum biological sample as a biomarker of sepsis in a human subject.

5. The in vitro use of any of claims 1 to 3, wherein the sepsis is the host response to a pathogen infection.

6. An *in vitro* diagnostic method for determining whether a subject, preferably a human subject, suffers or not from sepsis, that comprises the following steps:
a) Measuring or detecting the nuclease activity of the canonical RNases pertaining to the RNAse A family of endoribonucleases, in an isolated blood or serum sample obtained from said subject; and
b) comparing the measurement of the nuclease activity detected in (a) to a control reference or to a reference value, or value range, or to the value, or value range, obtained from an uninfected sample,
wherein such comparison shall indicate whether the subject does or not suffer from sepsis based on the RNase activity detected in the isolated sample.

7. An *in vitro* diagnostic method for determining whether a subject suffers or not from sepsis that comprises
a. Lysing a blood sample isolated from said subject allowing the release of the Blood ERythrocyte-derived RNase Inhibitor Activity (BERRIA);
b. Measuring or detecting the nuclease activity of the canonical RNases pertaining to the RNAse A family of endoribonucleases, in said sample after the lysing step a);
c. comparing the measurement or detection of the nuclease activity detected in (b) to a control reference or to a reference value or to the value obtained from an uninfected sample,
wherein
such comparison shall indicate whether the subject does or not suffer from sepsis based on the RNAse activity detected in the isolated sample.

8. The diagnostic method according to any of claims 6 or 7, wherein the sepsis is caused by a microbial, viral or fungi infection.

9. The *in vitro* diagnostic method of any of claims 6 to 8, wherein said method comprises measuring or detecting the fluorescence of the sample after contacting or incubating said sample with at least one probe comprising an oligonucleotide of 5- 30 nucleotides in length comprising a cleavage region between 1 to 3 RNA pyrimidines susceptible of being cleaved by any of the canonical RNases pertaining to the RNAse A family of endoribonucleases, a fluorophore operably linked to the oligonucleotide, and a quencher operably linked to the oligonucleotide, wherein the fluorescence level shall indicate whether the subject does or not suffer from sepsis.

10. The method according to claim 9, wherein the oligonucleotide contains at least one chemical modification.

11. The method according to claim 10, wherein the at least one chemical modification is preferably at the 2'position of the sugar ribose, preferably selected from the list consisting of 2'-O-methyl, 2'-fluoro, 2'-O-propargyl and/or LNA (locked nucleic acids).

12. The method according to any of claims 6 to 11, wherein the probe comprising an oligonucleotide is selected from the list consisting of SEQ ID: 1 to SEQ ID: 12

13. The method according to claim 12, wherein the probe comprising an oligonucleotide is SEQ ID: 7 or any of SEQ ID NO 1 to 3.

14. The *in vitro* diagnostic method of any of claims 6 or 7, wherein said method comprises detecting the nuclease activity, preferably visually, by using a lateral flow device.

15. The method according to claim 14, wherein said lateral flow device comprises:
(a) at least one probe comprising an oligonucleotide of 5- 30 nucleotides in length comprising a cleavage region between 1 to 3 RNA pyrimidines susceptible of being cleaved by any of the canonical RNases pertaining to the RNAse A family of endoribonucleases, wherein the oligonucleotide sequence is **characterized by** comprising a capture tag and a reporter molecule at each end of the sequence, for determining whether a subject suffers or not from sepsis;
(b) a support suitable for lateral flow that comprises:
b1. a backing card; and
b2. a sample pad, a membrane and a wicking pad, all of them on top of the backing pad, wherein the membrane is located after the sample pad so that the sample flows from the sample pad to the membrane, wherein the membrane comprises at least two test lines which in turn comprises molecules immobilized in the test line for capturing the capture molecule of the oligonucleotide sequence and wherein the wicking pad is located at the end of the membrane.

16. The method according to claim 15, wherein said lateral flow device comprises:
(a) the probe with any of SEQ ID NO.: 1-3, or 7 **characterized by** comprising a capture tag and a reporter molecule at each end of the sequence, for determining whether a subject suffers or not from sepsis;
(b) a support suitable for lateral flow that comprises:
b1. a backing card; and
b2. a sample pad, a membrane and a wicking pad, all of them on top of the backing pad, wherein the membrane is located after the sample pad so that the sample flows from the sample pad to the membrane, wherein the membrane comprises at least a test line which in turn comprises molecules immobilized in the test line for capturing the capture molecule of the SEQ ID NO.: 7 and wherein the wicking pad is located at the end of the membrane.

17. The method according to claim 14, wherein said lateral flow device comprises:
(a) at least one probe comprising an oligonucleotide of 5- 30 nucleotides in length comprising a cleavage region between 1 to 3 RNA pyrimidines susceptible of being cleaved by any of the canonical RNases pertaining to the RNAse A family of endoribonucleases, wherein the oligonucleotide sequence is **characterized by** comprising two capture tags at each end of the sequence, wherein each capture tag is different from the other and wherein one of the capture tags is capable of binding a reporter molecule, for determining whether a subject suffers or not from sepsis;
(b) a support suitable for lateral flow that comprises:
b1. a backing card; and
b2. a sample pad, a membrane and a wicking pad, all of them on top of the backing pad, wherein the membrane is located after the sample pad so that the sample flows from the sample pad to the membrane, wherein the membrane comprises at least two test lines which in turn respectively comprises molecules immobilized in each test line for capturing the capture tag of the oligonucleotide sequence, wherein the wicking pad is located at the end of the membrane,
and wherein the system further comprises a conjugate pad between the sample pad and the membrane, which in turn comprises a reporter molecule.

18. The method according to claim 17, wherein said lateral flow device comprises:
(a) the probe with any of SEQ ID NO.: 1-3, or 7 **characterized by** comprising two capture tags at each end of the sequence, wherein each capture tag is different from the other and wherein one of the capture tags is capable of binding a reporter molecule, for determining whether a subject suffers or not from sepsis;
(b) a support suitable for lateral flow that comprises:
b1. a backing card; and
b2. a sample pad, a membrane and a wicking pad, all of them on top of the backing pad, wherein the membrane is located after the sample pad so that the sample flows from the sample pad to the membrane, wherein the membrane comprises at least two test lines which in turn respectively comprises molecules immobilized in each test line for capturing the capture tag of the probe with SEQ ID NO.: 7, wherein the wicking pad is located at the end of the membrane,
and wherein the system further comprises a conjugate pad between the sample pad and the membrane, which in turn comprises a reporter molecule.

19. The method according to any of claims 14 to 18, wherein the lateral flow device further comprises a control line.

20. The method of claims 6 or 7, wherein the detection of the activity of the RNAse A family of endoribonucleases is performed by nuclear magnetic resonance (NMR).

21. The method of claims 6 or 7, wherein the detection of the activity of the RNAse A family of endoribonucleases is performed by solid support methods that facilitates immunoassay techniques such as enzyme linked immuno-sorbent assay (ELISA), a radioimmunoassay (RIA), an immuno radiometric assay (IRMA), a fluorescent immunoassay (FIA), a chemiluminescent immunoassay (CLIA), magnetic beads-based immunoassay (MBI), or an electro-chemiluminescent immunoassay (ECL).
